# EUROPEAN PATENT SPECIFICATION

(11) **EP 2 071 027 B1**
(45) Date of publication and mention of the grant of the patent: **26.10.2011**
(21) Application number: 07024062.7
(22) Date of filing: 12.12.2007
(51) Int. Cl.: C12N 15/09

(54) **Method of generating Rho° cells**
Verfahren zur Erzeugung von Rho°-Zellen
Procédé pour la génération de cellules Rho°

(43) Date of publication of application: 17.06.2009
(73) Proprietor: Mitogenomix GmbH, 97292 Uettingen (DE)
(72) Inventor: SEIBEL, Peter, 97292 Uettingen (DE)
(74) Representative: Schüssler, Andrea

(56) References cited:
- WO-A-99/55845
- KING M P AND ATTARDI G: "Human cells lacking mtDNA: Repopulation with exogenous mitochondria by complementation" SCIENCE, WASHINGTON, DC, vol. 246, 1 January 1989 (1989-01-01), pages 500-503, XP002131639 ISSN: 0036-8075
- MARUSICH M F ET AL: "Expression of mtDNA and nDNA encoded respiratory chain proteins in chemically and genetically-derived Rho0 human fibroblasts: a comparison of subunit proteins in normal fibroblasts treated with ethidium bromide and fibroblasts from a patient with mtDNA depletion syndrome" BIOCHIMICA ET BIOPHYSICA ACTA. MOLECULAR BASIS OF DISEASE, AMSTERDAM, NL, vol. 1362, no. 2-3, 31 December 1997 (1997-12-31), pages 145-159, XP004276729 ISSN: 0925-4439

## Description

The present invention relates to a method of the generation of 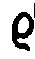 ⁰ cells using a mitochondrial targeted restriction endonuclease.

Mitochondria are organelles that can be found in most eukaryotic cells. Mitochondria harbour critical biochemical processes, such as the Krebs cycle or the aerobic energy supply of the cell. More recently, however, it was shown that they are key-players in the ageing process and the programed cell death. One factor closely linked to all these functions is the genome of the organelle, the mitochondrial DNA. Numerous neurological and neuromuscular diseases with a variety of symptoms have been associated with mutations of the mitochondrial genome (Wallace (2001) Novartis. Found Symp., 235, 247-263). Mitochondrial encephalopathies for example are a class of diseases which result from dysfunction of the mitochondria's oxidative phosphorylation system (OXPHOS). Under regular conditions, this system takes responsibility for cellular respiration and energy production. The associated diseases exhibit disorders manifesting in tissues with high aerobic metabolic demands (e.g. brain, skeletal muscle, heart). The oxidative phosphorylation system involves five enzyme complexes that assemble from subunits encoded by the nuclear DNA (nDNA) and the mitochondrial DNA (mtDNA). The mitochondrial genome is organized in a circular fashion, encompassing 16569 bp and encoding 13 polypeptides involved in OXPHOS, a set of 22 essential tRNAs as well as the large (16S) and small (12S) ribosomal RNA required for mitochondrial translation. Although the mtDNA was the first genome completely sequenced, almost nothing is known about the processes of how nucleus and mitochondria interact, of how mtDNA and nDNA gene expression is coordinated or how mtDNA is maintained within the cell (a process strictly driven by nuclear factors). Resulting from this lack of knowledge, very little is known about the molecular mechanisms leading to human diseases as a result of mtDNA damage. This is due, in part, to the inability two study regulatory and developmental mitochondrial processes under experimental conditions.

One effort to overcome these difficulties was an experiment carried out by King and Attardi (1989) Science., 246, 500-503. In resembling experiments known from yeast genetics, a human osteosarcoma cell line (143B.TK; derived from an osteosarcoma) was treated over 4 - 6 weeks with a low dosage of ethidium bromide. This reagent is known to interact with mtDNA to form complexes that interfere with DNA replication. After a series of cell divisions, the endogenous mtDNA is lost, while nuclear DNA is maintained. Since the loss of mtDNA extinguishes the oxidative phosphorylation system after a few days, cells devoid of endogenous mtDNA (so called 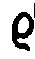 ⁰ (rho⁰ cells) need supplementation with nutrients to sustain viability. This is achieved by adding pyruvate and uridine to the growth medium so that the energy demand of the cell is satisfied additionally to the possibility of generating pyrimidines in spite of the inhibition of dihydroorotate dehydrogenase (DHODH) due to loss of respiratory chain electron transfer.

Additionally, other reagents were tested to generate 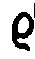 ⁰cells that interfere with mtDNA replication (ditercalinium, ddC, etc.). However, all chemicals used presented severe disadvantages, such as mutagenic effects or the induction of mdr (multi drug resistance) family gene expression so that the inhibitory effect on mtDNA replication was abolished. Moreover, the major limitation of these methods is their inapplicability to different cell lines. For example, despite anecdotal evidence of many attempts, there are no published accounts of rat 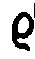 ⁰cell lines produced by these methods.

Human Molecular Genetics, vol. 14, no. 7, pages 893-902, 2005 relates to the effect of deletions by double-strand breaks in muscle of transgenic mice and describes the transfection of mtDNA containing cells with a vector comprising a sequence encoding a mitochondrial targeting sequence linked to the bacterial Pst I.

Further, mtDNA depletion *in vitro* using a restriction endonuclease linked to a mitochondrial targeting sequence is described in Gene Therapy, voL 14, pages 1309-1318, 2007 and Biomed Sci., vol. 9, pages 534-541, 2002. The selective destruction of mutant but not wild-type mtDNA which allows repopulation of wild-type DNA is described. This mtDNA depletion is modulated through the mtDNA heteroplasmy of the targeted cells.

Thus, the technical problem underlying the present invention is to provide a method for the generation of 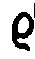 ⁰cells overcoming the disadvantages of the methods of the prior art.

The solution to the above technical problem has been achieved by providing the embodiments characterized in the claims. The present inventors developed a strategy based on a restriction endonuclease targeted to the matrix of mitochondria that allows the destruction of all endogenous mtDNA. The mtDNA is cleaved by the restriction endonuclease and endogenous nucleases act to fully disintegrate the mtDNA. This strategy was realized by selecting the gene for the restriction endonuclease EcoRI that is known to cleave human mtDNA approximately 3 - 5 times. By fusing the EcoRI gene to a mitochondrial targeting sequence derived from the gene of subunit VIII of human cytochrome c oxidase (COX VIII) and to the gene of EGFP (enhanced green fluorescent protein) as optical marker, mitochondrial localization was achieved. After transfection, selection with either geneticine or FACS analyses and growth of a cell clone designated as 143B.TK-K7, the loss of all endogenous mitochondrial DNA was confirmed by metabolic testing, PCR and Southern blot analyses. Additional comparative studies were carried out to characterise the proliferative, metabolic and morphological changes due to the 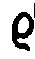 ⁰ state of these cells.

After derivation of the 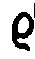 ⁰ clone 143B.TK-K7, the EGFP fluorescence of the EcoRI fusion protein could not be detected further. Therefore it was tested by PCR if the cells had lost the gene for the restriction endonuclease construct after destruction of the mtDNA or if some gene parts were integrated into the nuclear genome so that continually low amounts of the restriction endonuclease were expressed that would render the cell line useless as acceptor cell line in cytoplast fusion experiments. Analyses with different primer pairs amplifying varying regions of the EcoRI gene showed that no amplification products could be observed. Obviously, the gene for EcoRI was not Integrated into the nuclear genome of the 143B.TK-K7 cells. These results prove that only a short temporal expression of the restriction endonuclease targeted to the micochondria is sufficient to destroy completely, irreversibly and stably the mitochondrial genome, also indicating that DNA-repair systems in mitochondria are not efficient enough to cope with the DNA damage.

Finally, a 143B.TK- 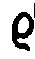 ⁰ cell line generated by incubation with low doses of ethidium bromide and the newly developed 143B.TK-K7 cell clone were compared to wild type cells for growth rate, glucose consumption as well as lactate and proton production rate.

All generated culture cells depleted of mtDNA so far proliferate despite their lack of oxidative phosphorylation in mitochondria. However, 143B.TK cells with 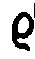 ⁰ genotype exhibit a decrease in proliferation rate compared to wild type. This feature was apparent in the newly established 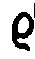 ⁰ cell line 143B.TK-K7 due to the exclusive anacrobic energy production via glycolysis. Thus, all 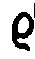 ⁰ cell lines depend on supplementation with pyruvate and uridine. Additionally, they show an increased acidification of the culture medium by lactic acid (Jakobs et al. (1994) Biochim. Biophys. Acta., 1211, 37-43). Within this work the dependence on supplementation with pyruvate and uridine was also obvious for the 143B.TK-K7 cells under normal growth conditions as well as an acidification of the culture medium by lactic acid fermentation.

Investigation of the culture medium showed that the pH value of the 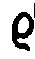 ⁰cells was substantially decreased, compared to wild type medium. While it was not possible to determine the 62 hours value of the 143B.TK- cells due to cell mortality that was caused by high cell density, cells depleted of mtDNA exhibited a further decline of the pH to 6.7 - 6.8. Standardised to 10⁶ cells, the glucose consumption as well as the lactate and proton production of the 143B.TK-K7 and 143B.TK- 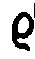 ⁰ cells were increased. In contrast to the wild type cells, this results from a complete dependence of the 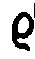 ⁰ cells on glycolysis for ATP production and therefore an acidification by enhanced lactic acid fermentation occurs. It was shown that in MOLT-4 cells the lactate production was increased 4-fold compared with the wild type (Armand et al. (2004) Toxicol. Appl. Pharmacol, 196, 68-79). Though in the present work the lactic acid production of the cells depleted of mtDNA was only increased 1.5 - 2-fold in contrast to the wild type.

The transport of lactate and other monocarboxylates within and between cells is accomplished by a family of monocarboxylate transport proteins (MCTs). The mitochondrial lactate/pyruvate exchanger probably collaborates with a mitochondrial lactate dehydrogenase that enables the oxidation of lactate in active respiring cells ( Brooks (2002) Biochem. Soc. Trans., 30, 258-264). This pathway can not take place in 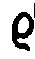 ⁰ cells, so that the total amount of lactate produced must be released into the surrounding medium. Across the plasma membrane, lactate is co-transported with protons (Poole and Halestrap (1993) Am. J. Physiol., 264, C761-C782) accounting for the decrease in pH value of the culture medium in the 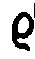 ⁰ cells.

The morphology of the mitochondria in the denoted cell lines was studied with confocal microscopy using MitoTracker Red CMXRos to stain the organelles. The observations of a widespread mitochondrial network in wild type cells in contrast to punctated mitochondria which often seem to be swollen in 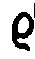 ⁰ cells constitute typical changes of mitochondrial morphology due to a loss of mitochondrial genomes. Additionally, the total amount of mitochondrial volume seemed not to be altered between the normal and 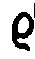 ⁰ cells. Even more morphological changes of the 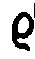 ⁰mitochondria were detected by ultrastructural studies. In contrast to the mitochondrial reticulum in normal 143B.TK- cells with electron dense matrix full of regular arranged cristae structure, the TEM images of the 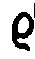 ⁰ cells show an apparent altered morphology of the mitochondria. The network is degraded in single mitochondrial units often with swollen appearance and electron empty matrix probably due to more dilution by increasing influx of water. Most cristae exist as "fuzzy onions"-like structures (Hales and Fuller (1997) Cell., 90, 121-129) composed of two membranes lying in concentric rings in the matrix Additionally, these membranous rings showed only few contact sites with the mitochondrial border membrane. It is not clear whether these double membranes consist of two inner membranes or one outer and one inner membrane.

Similar 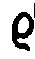 ⁰ phenotypes with swollen mitochondria with membranous inclusions and multiple concentric cristae could be observed after incubating cells with the reverse transcription inhibitor zidovudine (Semino-Mora et aL (1994) Lab Invest, 71, 773-781). However, by addition of L-carnitine these alterations could be avoided. This indicates a probable involvement of defective fatty acid pathways in these morphological changes.

By applying the new method described here it is possible for the first time to develop new 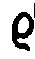 ⁰ cell lines under very controlled and mild conditions, avoiding mutagenic effects of chemicals (e.g. ethidium bromide). Thus, 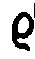 ⁰ cell lines exhibiting varying nuclear backgrounds can be used in future experiments to study diseases connected to mitochondrial phenotypes.

### Brief description of the drawings

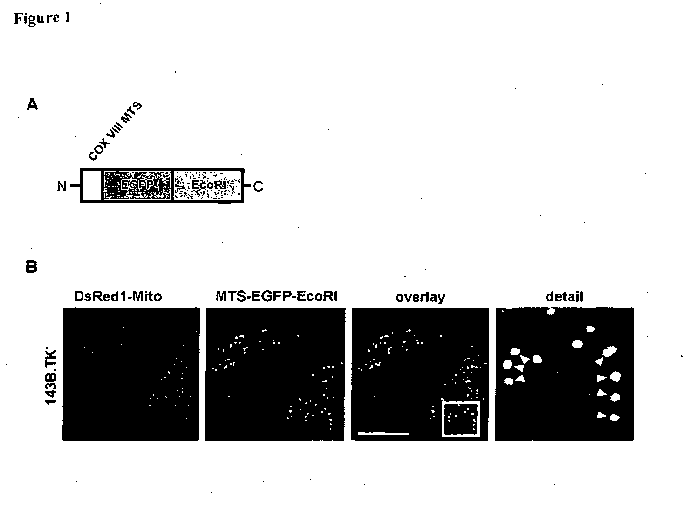

Figure 1: Construction and expression of the mitochondrially targeted EcoRI
   A DNA fragment coding for the COX VIII targeting sequence (MTS) was added to the 5' end of the restriction endonuclease gene EcoRI and as "optical" marker the gene for the enhanced green fluorescent protein EGFP was fused to the 3' end. The construct was subcloned into the vector pTRE2hyg and recloned with AgeI and NotI into pEGFP-Mito resulting in the vector pMEE-con with a constitutive CMV promoter **(A).** The final construct was transfected in 143B.DsRed1-Mito cells. Twenty-four hours after transfection the mitochondrial localisation of the EcoRI fusion protein in punctate structures (white arrowheads) was confirmed by colocalisation with DsRed1-Mito with confocal fluorescence microscopy **(B).** The calibration mark corresponds to 10 µm.
Figure 2: Graphic documentation of the proliferation rate of the cell lines 143B.TK-K7 (dotted line), 143B.TK- 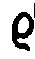 ⁰ (solid line) and 143B.TK- (dashed line) at proceeding cultivation times
   The indicated values are averages of four individual measurements. The standard deviations are depicted as error bars. The 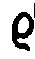 ⁰ cells exhibit a decelerated proliferation rate compared to the wild type.
Figure 3: Depletion of mtDNA in the cell line 143B.TK-K7 after expression of a mitochondrially targeted EcoRI
   143B.TK- wild type and 143B.TK- 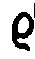 ⁰ controls as well as the isolated 143B.TK-K7 cells were analysed by PCR using primers corresponding to the D-loop region of mtDNA additionally to primers amplifying a histon H1 of nuclear DNA. It was not possible to amplify this mtDNA region in the cell lines 143B.TK- 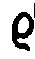 ⁰ and 143B.TK-K7 confirming the depletion of the mitochondrial genome in these cells. Positive amplification of histone H1 shows a sufficient amount of genomic DNA in all probes **(A).** Southern blot analyses with these cells after digestion with BamHI or PvuII using a probe coding for mtDNA nucleotides 4831-5651 also showed the absence of mtDNA in the 143B.TK- 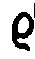 ⁰ and 143B.TK-K7 cells **(B).**

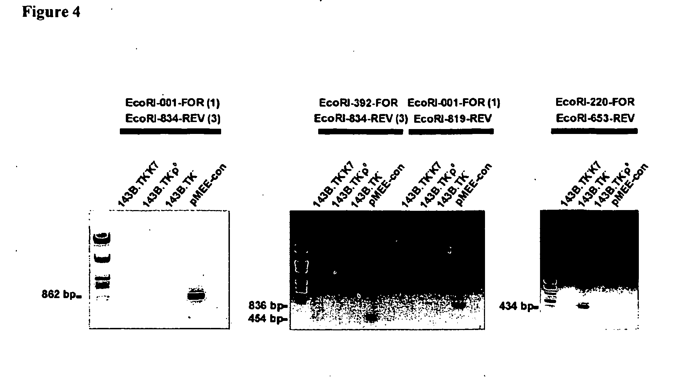

Figure 4: PCR analyses show no integration of the gene coding for EcoRI into the nuclear genome of 143B.TK-K7 cells
   PCR analyses were carried out using different primer pairs as indicated amplifying various regions of the EcoRI gene in the cell lines 143B.TK-K7, 143B.TK- 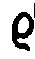 ⁰, 143B.TK- and with 50 pg pMEE-con as positive control. The results depict no integration of the restriction endonuclease gene into the genomc.

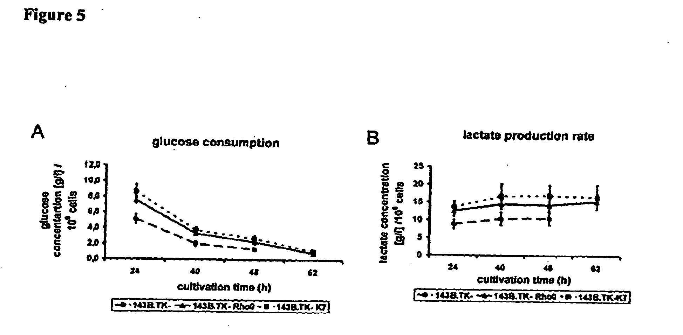

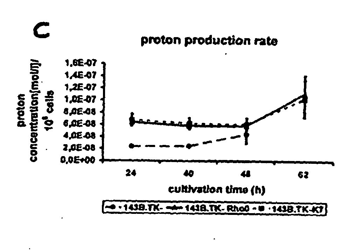

Figure 5: Graphic documentation of the glucose consumption **(A),** lactate production rate **(B)** and proton production rate **(C)** in pure culture medium (dash-dotted line) and the culture medium of the cell lines 143B.TK-K7 (dotted lines) 143B.TK- 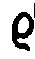 ⁰ (solid lines) and 143B.TK-(dashed lines) at proceeding cultivation times standardised to 10⁶ cells
   The indicated values are averages of four individual measurements respectively eight independent measurements (pH value of 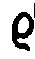 ⁰cells). The standard deviations are depicted as error bars. Standardised to 10⁶ cells the glucose consumption as well as the lactate and proton production of the 143B.TK-K7 and 143B.TK- 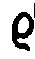 ⁰ cells exceed the amounts of the 143B.TK- wild type. However, the pH value of culture medium in all determined cell lines was nearly similar. The pH value of pure medium without addition of cells shows a slight decline due to saturation of medium with CO₂. An especial curve progression could be observed in the analysis of proton production rate where the values initially decline and then increase. It was not possible to determine the 62 hours cultivation value of the wild type cells because of extreme cell density resulting in apoptotic events.

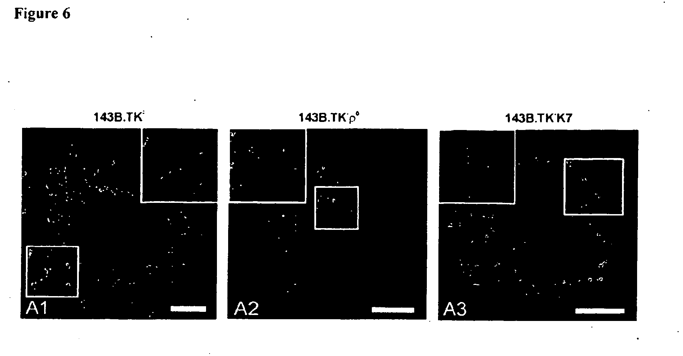

Figure 6: Mitochondrial organisation in wild type and 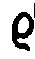 ⁰ 143B.TK- cells
   The cells were stained with MitoTracker Red CMXRos. A1 shows the predominantly reticular organization of mitochondria in wild type cells with mainly rod shaped organelles. 143B.TK- 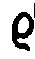 ⁰ (A2) and 143B.TK-K7 cells (A3) stained with MitoTracker Red CMXRos denotes the disruption of the mitochondrial reticulum in single units that often seemed to be swollen. Calibration marks correspond to 10 µm.

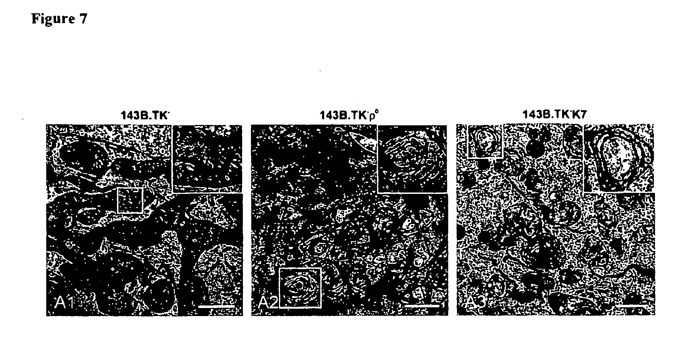

Figure 7: Ultrastructural investigation of wild type and 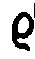 ⁰ mitochondria by TEM
   Electron micrographs of ultrathin sections are shown. 143B.TK- wild type mitochondria show an interconnected network structure with numerous regular arranged cristae (A1). The mitochondria of 143B.TK- 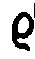 ⁰ (A2) and 143B.TK-K7 cells (A3) demonstrate single vesicular organelles with distorted cristae, lying in concentric double membrane rings in the matrix. The insets show higher magnifications of the boxed areas. Bars (A1-A3), 1 µm.

Thus, the present invention provides a method of the generation of a 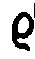 ⁰ cell comprising the following steps: (a) transfecting mtDNA containing cells with an expression vector containing a gene encoding a fusion protein comprising a mitochondrial targeting sequence (MTS) and a restriction endonuclease operatively linked to a suitable promoter wherein the restriction endonuclease cleaves the not DNA 3 to 10 times, (b) culturing the transfected cells over a sufficient period of time; and (c) selecting 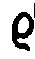 ⁰ cells.

There are various methods of introducing foreign DNA into a prokaryotic or eukaryotic cell available that are well known to the person skilled in the art, e.g., transfection by calcium phosphate. Other methods of transfection include electroporation, heat shock, magnetofection or are based on the use of proprietary transfection reagents such as Lipofectamine^{™}, Fugene^{™}, jetPEI™, Effectene^{™} or DreamFect^{™}. The recombinant (expression) vectors (as well as genes encoding a fusion protein) can be constructed according to methods well known to the person skilled in the art; see, e.g., Sambrook (1989), Molecular Cloning A Laboratory Manual, Cold Spring Harbor Laboratory N.Y. A variety of expression vectors, preferably expression vectors for stable expression, may be utilised to contain and express sequences encoding the fusion protein of the present invention. These include recombinant bacteriophages, plasmids, or cosmid DNA expression vectors, e.g., yeast expression vectors, virus expression vectors (e.g., baculovirus); plant cell expression vectors (e.g., cauliflower mosaic virus, CaMV; tobacco mosaic virus, TMV), bacterial expression vectors (e.g., Ti or pBR322 plasmids) or animal expression vectors, e.g., vectors based on retroviruses, lentiviruses, adenoviruses or adeno-associated viruses. Moreover, for transfection nanoengineered substances may be used.

Methods for the cultivation of cells and appropriate culture media are known to the skilled person and depend on the kind of cell to be cultivated. The term "culturing the transfected cells over a sufficient period of time" means cultivation allowing the complete destruction of the mtDNA, i.e., expression of the foreign gene, i.e., synthesis of the fusion protein, delivery of the fusion protein into the mitochondria and cleavage of the mtDNA by the restriction enzyme being a part of the fusion protein. The time that is sufficient for achieving this aim depends on various factors, e.g., the kind of vector and promoter used, the particular cultivation conditions and the kind of cells but can be easily determined by the skilled person using established assays, e.g., the assays described in the examples, below. For animal cells, a "sufficient period of time" might be in the range of 12 hours to 20 days.

In the method of the present invention, the restriction endonuclease is a so-called "rare cutter" that cleaves the mtDNA 3 to 10 times, preferably 3 to 7 times. Such restriction enzymes are well known to the person skilled in the art and genes encoding these enzymes have been published and can, thus, be used for constructing a fusion protein additionally comprising a mitochondrial target sequence. Examples of preferred restriction enzymes are AflII, BamHI, BclI, EcoRI, HaeIII, HindII, HindIII, NdeI, PvuII and SpeI.

The method of the present invention is generally applicable to any cell containing mitochondria, i.e., any eukaryotic cell. Preferred eukaryotic cells are animal cells. Mammalian cells, e.g., monkey, rat, guinea pig, porcine or human cells are particularly preferred.

To express the inserted gene encoding a fusion protein comprising a mitochondrial targeting sequence (MTS) and a restriction endonuclease operatively linked to a suitable promoter, it is preferable to use a strong eukaryotic promoter such as the CMV immediate early (IE) promoter, the Rous sarcoma virus (RSV) LTR, the Mouse mammary tumor viral promoter with Dioxane response elements, the β-Actin gene promoter; the Metallothionin promoter with NRE or GRE elements.and the SV40 virus early promoter. The CMV IE promoter can be the human promoter (HCMV IE) or the murine promoter (MCMV IE), or alternatively a CMV IE promoter of another origin, for example of monkey, rat, guinea pig or porcine oxigin. In general, a promoter for constitutive expression is preferable.

In a preferred embodiment of the method of the present invention, the expression vector is a vector for animal cells. Suitable vectors are well known to the skilled person and examples of such vectors have already be given above. Particularly preferred animal vectors are human papova viral based vectors (BKV), SV40 derived vectors, vaccinia derived vectors, adeno viral derived vectors, baculo viral vectors, or retroviral derived vectors. Alternatively, non-viral mammalian vectors such as TRE2hyg can be employed.

Proteins are targeted to submitochondrial compartments (the outer membrane, intermembrane space, inner membrane and matrix) by multiple signals and several pathways. Most mitochondrial proteins are synthesized as cytosolic precursors containing uptake peptide signals. Cytosolic chaperons deliver preproteins to channel linked receptors in the mitochondrial membrane. The preprotein with a presequence targeted for the mitochondria is bound by receptors and the General Import Pore (GIP) (Receptors and GIP are collectively known as Translocase of Outer Membrane or TOM) at the outer membrane. The preprotein is translocated through TOM as bairpin loops. The preprotein is transported through the intermembrane space by small TIMs (which also acts as molecular chaperones) to the TIM23 or 22 (Translocase of Inner Membrane) at the inner membrane. Within the matrix the targeting sequence is cleaved off by mtHsp70. Three mitochondrial outer membrane receptors arc known: TOM20, TOM22 and TOM70. TOM70 binds to internal targeting peptides and acts as a docking point for cytosolic chapetopes. TOM20 binds presequences and TOM22 binds both presequences and internal targeting peptides The TOM channel is a cation specific high conductance channel with a molecular weight of 410 kDa and a pore diameter of 21Å. The presequence translocase 23 (TIM23) is localized to the mitochondial inner membrane and acts a pore forming protein which binds precursor proteins with its N-terminal. TIM23 acts a translocator for preproteins for the mitochondrial matrix, the inner mitochondrial membrane as well as for the intermembrane space. TIM50 is bound to TIM23 at the inner mitocondrial side and found to bind presequences. TIM44 is bound on the matrix side and found binding to mtHsp70. The presequence translocase22 (TIM22) binds preproteins exclusively bound for the inner mitochondrial membrane. Mitochondrial matrix targeting sequences are rich in positively charged amino acids and hydroxylated ones.

Mitochondrial target sequences (MTS) suitable for the method of the present invention are well known to the person skilled in the art. MTS to the mitochondrial matrix are preferred and a preferred member of such MTS is a targeting peptide from the cytochrome c oxidase subunit 4 (COX IV) or subunit 8 (COX VIII). In principle, any target sequence derived from any nuclear encoded mitochondrial matrix or inner membrane enzyme or an artificial sequence that is capable of rendering the fusion protein into a mitochondrial imported protein (hydrophobic moment greater than 5.5, at least two basic residues, amphiphilic alpha-helical conformation; see Bedwell et al., Mol Cell Biol. 9(3) (1989), 1014-1035) is useful for the purposes of the present invention.

In an even more preferred embodiment of the method of the present invention, the fusion protein furthermore comprises a detectable polypeptide, preferably a fluorescence protein, allowing (a) to monitor the expression of the gene encoding the fusion protein in the cells and/or (b) to carry out FACS analyses so that transfected cells can be selected for rho⁰ cells. Suitable fluorescence proteins are known in the art such as Reef Coral Fluorescent proteins, AmCyan Fluorescent Protein, Asked Fluorescent Protein, DsRed Fluorescent Protein, HcRed Fluorescent Protein, ZsGreen Fluorescent Protein, ZsYellow Fluorescent Protein, GFP, YFP, CFP, EGFP. Examples of preferred fluorescence proteins are GFP and EGFP. Due to the potential for widespread usage and the evolving needs of researchers, many different mutants of GFP are in the meantime available. The first major improvement was a single point mutation (S65T) that dramatically improved the spectral characteristics of GFP, resulting in increased fluorescence, photostabliliry and a shift of the major excitation peak to 488 nm with the peak emission kept at 509 nm. The addition of the 37°C folding efficiency (F64L) point mutant to this scaffold yielded enhanced GFP (EGFP). Superfolder GFP, a series of mutations that allow GFP to rapidly fold and mature even when fused to poorly folding peptides, was reported in 2006. Many other mutations have been made, including color mutants; in particular blue fluorescent protein (EBFP, EGFP2, Azuritc), cyan fluorescent protein (ECFP, Cerulean, CyPer) and yellow fluorescent protein derivatives (YFP, Citrine, Venues, YPet). BFP derivatives contain the Y66H substitution. The critical mutation in cyan derivatives is the Y66W substitution, which causes the chromophore to form with an indole rather than phenol component A red-shifted wavelength of the YFP derivatives is accomplished by the T203Y mutation. All of these fluorescence proteins can be used for the method of the present invention.

The person skilled in the art also knows methods for analysing 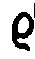 ⁰ cells and for separating 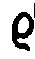 ⁰ cells from 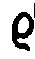 ⁻ or 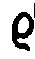 ⁺ cells. In a further preferred embodiment of the method of the present invention the rho0 cells are selected by FACS analysis, tissue culture cloning techniques (cloning ring method, cell dilution method etc), metabolic testing (growing on uridine/pyruvate supplemented medium, while dying on uridine/pyruvate depleted growth medium) or genetic testing, for example by PCR or Southern Blot analysis.

The below examples explain the invention in more detail.

### Example 1

### Materials and Methods

### (A) Cloning

Cloning was performed according to standard procedures, and all polymerase chain reaction (PCR) products were versified by sequencing. The plasmid pAN4 (a kind gift of A- Kiss and constructed by P. Modrich and coworkers, Newman et al. (1981) J. Biol. Chem., 256, 2131-2139.) coding for the EcoRI gene was used as template in a PCR with following primers: EcoRIR-001-FOR (Anderson et al. (1981) Nature, 290, 457-465) 5'-catggacgagecgtacaagatgtctaataaaaaac-3', which adds a short sequence complementary to the 3'-end of the EGFP gene and EcoRIR-834-REV (Anderson et al. (1981) Nature, 290, 457-465) 5'-ggccaaatcacttagarguagctgttcaaac-3' which generates a Nod-restriction site. The EGFP gene from the plasmid pEGFP-Mito (Clonetech Europe, Saiot-Germain-en-Laye, France) was amplified with the primers prGFP-Mtito-0597-FOR 5'-ggccaaatgtccgtectgacg-3' which generates a Nod-restriction site and pEGFP-Mito-1421-REV 5'-cdgtacagctcgtccatgccg-3' which amplifies the EGFP without the stop-cndon. Both fragments were utilised in a recombinant PCR and the phosphorylated product was subcloned in the PvuII-lincatized vector pTRE2hyg (Clonetech Europe, Saint-Gennain-en-Laye, France). The fusion gene product consisting of EGFP- and EcoRI-gene was cloned with Agel and NotI into pEGFP-Mito resulting in the vector pMEE-con.

### (B) Cell Culture, Transfection and Mitochondrial Staining

Human osteosarcoma cells 143B.TK- (ATCC CRL-8303) were cultured under standard conditions in Dulbecco's modified Eagle's medium (high glucose) with Glutamaxx (Invitrogen, Karlsruhe, Germany) supplemented with 10 % fetal calf serum, 1% bromdeoxyuridine, 100 units penicillin and 100 µg/ml streptomycine. The 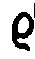 ⁰ cell line referred to as 143B.TK- 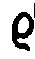 ⁰ was generated by incubation of 143B.TK- with low doses of ethidium bromide. 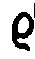 ⁰ cell lines of the parental 143B.TK- and cells after transfection procedure were maintained as indicated with additional supplementation with 100 µg/ml pyruvate and 50 µg ml uridine.

Transient transfections of 143B.TK- and 143B.DsRedl-Mito (143B.TK- cells stably expressing mitochondrially targeted DsRedl for co-localisation studies with pMEE-con) were performed using Effectene (Qiagen, Hilden, Germany) according to manufacturer's conditions. Geneticin selection with 700 µg/ml was carried out 24 h after transfection for 5 days. An isolated clone named as 143B.TK-K7 was aliquoted and metabolically tested for existing oxidative phosphorylation with DMEM (high glucose) with Glutamaxx, 10 % dialysed FCS, with 1 % bromdeoxyuridine, 100 units penicillin and 100 µg/ml streptomycine without pyruvate and uridine for 30 days.

Two other cell lines were used to extend the applicability of this approach to rodent cells: the mouse line LMTK- (ATCC CCL-1.3), and the rat line NRK52E (ATCC CRL-1571). MitoTraeker CMXRos (Invitrogen, Karlsruhe, Germany) was used to label mitochondria according to manufacrurer's protocol at a concentration of 100 nM.

### (C) FACS anises

Samples of transfected cells (2 x 10⁶ cells/ml in 0.05 % BSA in PBS) were analysed on a flow cytometer and its software (FACSCAN, FACS Vantage SE, Cell Quest Software, BD Biosciences). Acquisition of data was performed by gating more than 100.000 cells expressing the mitochondrial targeted restriction endonuclease fused to EGFP with the flow cytometer.

### (D) Characterisation of clones by PCR

To verify the loss of all endogenous mtDNA. in the resulting 143B.TK-K7 cell clone the genomic DNA was isolated and used as a template in a PCR. Controls were accomplished by using the genomic DNA isolated from the 143B.TK- wild type and a corresponding 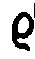 ⁰cell-line generated by incubation with ethidium bromide as described before. The following primers binding in the mtDNA region were utilized: 15501-FOR 5'-acccagacaattaiaccctagc-3' and 630 REV 5'-gagcccgtctaaacattttcaatg-3'. The control-PCR was performed with primers that amplify the human histone H1 gene (Histon H1 5' 5'-atgagctcatgaccgagaattccacgtccg-3' and Histon H1 3' 5'-accccgggcaaacttcttcugcc-3')- The annealing temperature was 55°C for 35 cycles with an elongation time of one minute.

To investigate a possible integration of the EcoRl gene into the genome of the 143B-TK-K7 cells a PCR was performed with several primer sets listed in the following, which amplify different regions of the EcoRI gene: EcoRI-001-FOR (Wallace (2001) Novartis Found. Symp., 235, 247-263.) 5'-aattcccggatcccaccatgtctaataaaaaacagtcaaa-3', EcoRJ-220-FOR 5'-gaccctgaccttggcggtactttatttg-3', EcoRI-392-FOR 5'-gaggagatcaagatttaatggctgctg-3', EcoRI-653-REV 5'-catagattactatttalaggcattccataattagctgc-3', EcoRI-819-REV 5'-ctgtteaaacaagtcacgcc-3', EcoRI-834-REV (King and Attardi (1989) Science., 246, 500-503) 5'-attcccccggg tcacttagatgtaagctgttca-3'. Annealing temperatures of the first 5 cycles were 45°C followed by 55 °C for 30 cycles with elongation times of 2 minutes.

### (R) Southern Blot Analysis

Total DNA (15 µg) extracted from cells was digested with the restriction endonucleases BamHI or PvuII. Restricted fragments were separated on a 0.6 % agarose gel, transferred onto a nylon membrane and hybridized with a digoxygenin-dUTP-labeled human mtDNA probe amplified with mtDNA primers annealing to nucleotides 4831-4846 (For) and 5628-5651 (Rev). The membrane was washed, and the fragments were detected with the DIG Nucleic Acid Detection Kit (Roche Applied Sciences, Mannheim Germany) according to the manufacturer's instructions. Chemoluminescence of the fragments was analysed with a bioimaging system (Alpha Imager, Biozym, Hessisch Oldendorf, Germany).

### (F) Metabolite Analyses of Culture Medium

4 x 10⁵ cells were seeded in a 35 mm dish and incubates in 2.5 ml culture medium supplemented with 100 µg/ml pyruvate and 50 µg/ml uridine under standard conditions. After 24, 40, 48 and 62 hours cultivation time the cell number was quantified, the pH value analysed and the glucose and lactatc; concentrations determine on a Hitachi 917 clinical chemistry analyser (Roche Diagnostics), The pH value was converted into proton concentration in medium by the equation proton conc (mol/l) = 10^{-p11}, standardised additionally to all other data to 10⁶ cells and analysed graphically- The indicated values are averages of four individual measurements or eight independent measurements (pH value of 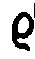 ⁰cells) respectively. Standard deviations are depicted as error bars.

### (G) Confocal Microscopy

Living cells cultured on glass bottom dishes (MatTek Corporation Ashland, USA) were observed with the inverted confocal laser scanning microscope TCS SP5 (Leica Microsystems). To avoid a cross talk in excitation of multiple stained compounds a sequential scanning mode was used in confocal microscopy exclusively. Images were acquired with photo multipliers and micrographs were processed and analysed with the software Leica Application Suite Advanced Fluorescence 1.5.1 and Adobe Photoshop CS.

### (H) Electron microscopy

Cells grown on cover slips were fixed with a solution of 2.5 % glutaraldehyde, 2 % formaldehyde (made from paraformaldehyde) in 100 mM cacodylate buffer, pH 7.4 for 1.5 h at 4 °C, washed twice with cacodylate buffer, followed by a fixation with 2% osmium tetroxid in 50 mM cacodylate buffer (pH 7.4). Specimens were washed twice with distilled water and stained over night with aqueous 0.5% uranyl acetate at 4°C. Cells were dehydrated and flat embedded in Epon 812. Ultrathin sections were analyzed with a Zeiss EM10 (Oberkochen, Germany). Negative were digitalized by scanning and processed with Adobe Photoshop CS.

**Example 2**

**Generation of** 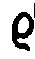 ⁰**cells utilizing** a **mitochondrial targeted restriction endonuclease and confirmation of** 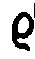 ⁰ **state**

The aim of this experiment was to develop a system for generating cells devoid of any endogenous mitochondrial DNA without using mutagenic substances like ethidium bromide, ditercalinum or 2'3'-dideoxycytidin. The basis of this system was the vector pMEE-con that after transfection in cells constitutively expresses the restriction endonuclease EcoRI as functional unit. EcoRI is known to cleave human mtDNA 3 - 5 times depending on the cells ethnic background. Laboratory mouse mtDNA also has 3 EcoRI sites, while Rattus norvegicus, the source of the NRK52E cell line, has 7 sites. EcoRI was fused to a mitochondrial targeting peptide derived from the human cytochrome c oxidase subunit 8 (COX VIII). Furthermore, this construct was fused to the enhanced green fluorescent protein EGFP (Fig.1A) on the one hand to observe the expression in the cells (Fig 1B) and to have a selection market suitable for FACS analysis on the other hand so that transfected cells can be selected to purity. Confocal fluorescence microscopy showed that the polypeptide was expressed and presented a mitochondrial localisation in punctate structure presumably in close contact to mtDNA. in nucleoids (Fig. 1B). After selection of transient transfected cells with the antibiotic geneticin for 5 days or alternatively by FACS selection, a clone of 143B.TK- could be isolated. This cell clone designated as 143B.TK-K7 was metabolically tested for its dependency on pyruvate and uridine. The cells exhibited only poor proliferation during cultivation in medium without pyruvate und uridine and finally died, strongly suggesting a 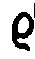 ⁰-state of the cell line.

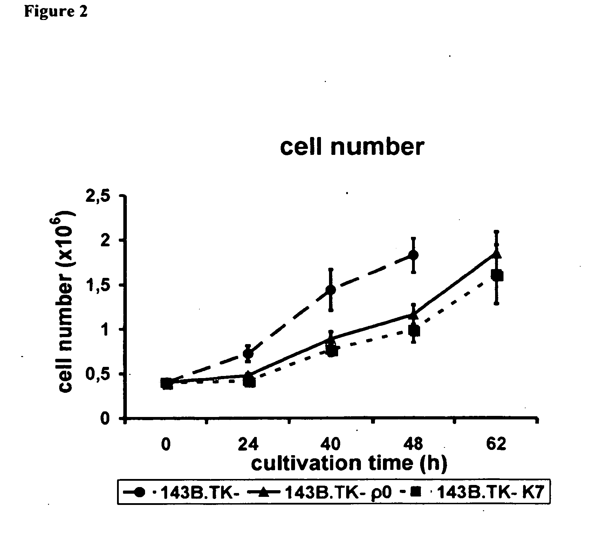

The analyses of cell proliferation during 62 hours of cultivation time (Fig. 2) showed an obvious decrease in proliferation rate of the 143B.TK-K7 cells (dotted lines) in contrast to the wild type (dashed lines). This is consistent to observations made with a 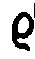 ⁰ cell line referred to as 143B.TK- 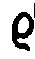 ⁰ generated by incubation with low doses of ethidium bromide (solid line). Both 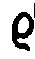 ⁰ cell lines exhibit a similar genetic background, however, the figure indicates that the 143B.TK-K7 cells posses a slightly lower growth rate than the 143B.TK- 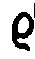 ⁰ cells.

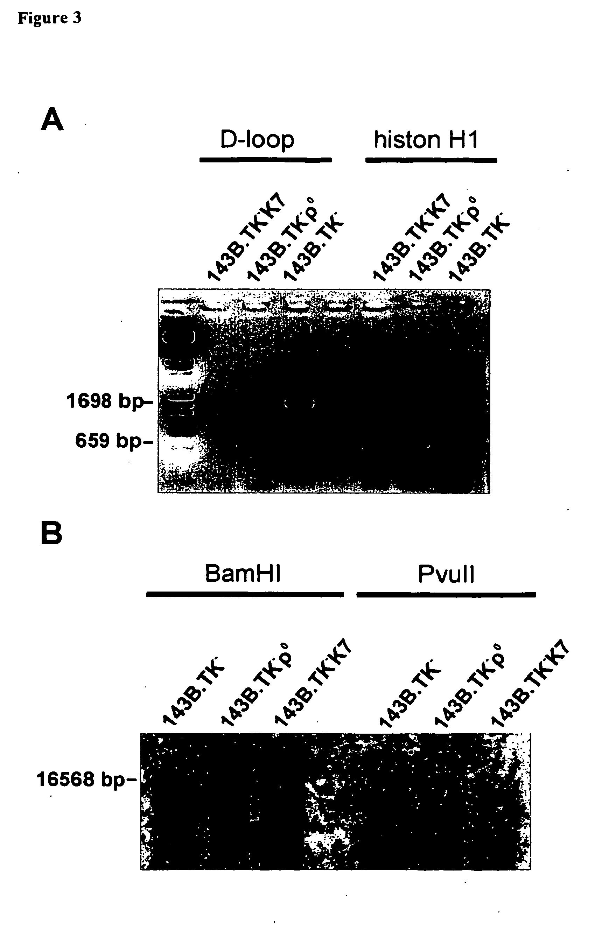

Additional confirmations of the depletion of mtDNA were carried out by PCR analyses on genomic DNA isolated from the 143B.TK-K7 cells with primers that implificare a 1698 bp region located within the D-loop (Fig. 3A). Positive controls for the amplification were carried out with genomic DNA of the wild type and as negative control of 143B.TK- 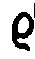 ⁰ cells. Additional examinations were accomplished with primers amplifying a region of the nuclear gene histone H1 to proof a sufficient amount of template DNA in the samples. In contrast to the wild type cell line, no amplification product could be observed in the 143B.TK- 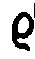 ⁰ and 143B.TK-K7 cells. Furthermore these results were additionally confirmed by Southern Blot testing and showed a complete loss of mtDNA in the 143B TK- 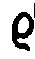 ⁰ and 143B.TK-K7 cells (Fig. 3B). Similar results were obtained in multiple independent clones of the LMTK- and NRK52E cell lines (data not shown).

### Example 3

### Analyses of integration of the restriction endonuclease gene into the genome

After passaging a stable 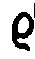 ⁰ clone 143B.TK-K7 the EGFP fluorescence of the EcoRI fusion protein expression was undetectable indicating that the cells had lost the plasmid. Then it was tested by PCR whether the cells had integrated the gene for the restriction endonuclease into their genome by amplifying various regions of the EcoRI gene. Therefore analyses with different primer pairs were carried out with genomic DNA (Fig.4). In the 143B.TK-K7 cells as well as in the controls no amplification products could be observed showing that the gene for EcoRI did not integrate into the nuclear genome of the 143B.TK-K7 cells. Furthermore, these results showed that only a short temporal expression of the restriction endonuclease targeted to the mitochondria is sufficient to destroy the mitochondrial genome completely. During more than one year of cultivation, there was no recover of mtDNA in the 143B-TK-K7 cells tested by PCR and metabolic dependence of pyruvate and uridine.

### Examples 4

### Comparative analyses of the culture medium

The energy production of 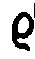 ⁰cells completely depends on anaerobic glycolysis. Additionally, all 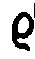 ⁰ cell lines known so fat exhibit a dependence on supplementation with pyruvate and uridine as well as an increasing acidification of the culture medium by excessive lactic acid production- Both characteristic features could be observed in the 143B-TK-K7 cells and the mouse and rat 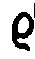 ⁰ clones. Therefore the 143B.TK-K7 cells were analysed after 24, 40, 48 and 62 hours of cultivation for glucose concentration, lactate concentration and pH value in the culture medium additionally to the determination of cell number. The glucose and lactate concentration as well as the proton concentration were standardised to 10⁶ cells and graphically illustrated. Reference values were obtained by the examination of 143B.TK- and 143B.TK- 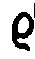 ⁰ cells. Standardised to 10⁶ cells, the glucose consumption (Fig. 5A) of the 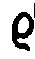 ⁰ cells (7.5 - 8.5 g/l, 143B.TK-0, solid line and 143B.TK-K7, dotted line) exceeds the amount of the wild type cells (5 g/l, dashed line). Furthermore it is obvious that the glucose consumption decreases during cultivation time (∼88 % in 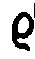 ⁰ cells and 75 % in wild type cells).

The graphical documentation of lactate production standardised to 10⁶ cells (Fig. 5B) shows a higher lactate production rate of the 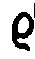 ⁰ cells (143B.TK- 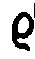 ⁰, solid line and 143B.TK-K7, dotted line) than the wild type. While the values initially increase during 24 until 40 hours cultivation, the curve progression resembles a saturation thereafter and then stays at an almost constant level (143B.TK-K7: 17 g/l, 143B.TK- 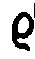 ⁰: 15 g/l, 143B-TK-: 11 g/l).

The pH values of pure medium without addition of cells show only a slight decline of pH 8.0 to pH 7.7 due to saturation of medium with CO₂ (Fig. 5C, dot-dashed line). On the other hand, the pH value of the medium of the examined cell lines changes from 7.8 in wild type cells (dashed line) and 7.5 in 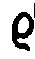 ⁰ cells (143B.TK- 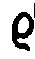 ⁰, sold line and 143B.TK-K7, dotted line) to pH 7.3 after 48 hours cultivation time in all cell lines (Fig. 5C). Whereas the 62 hour value of the 143B.TK- cells could not be determined due to cell mortality that was caused by high cell density, in the cells depleted of mtDNA the pH value further declined to 6.7 - 6-8.

The proton concentration standardised to the cell number at different cultivation times (Fig. 5D) exhibits that the proton discharging rate in the medium of 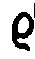 ⁰ cells (143B.TK- 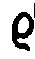 ⁰, solid line and 143B.TK-K7, dotted line) highly exceeds the demand of the wild type cells (25 - 3.5 x 10⁻⁸ mol/l, dashed line). Besides an especial curve progression could be observed in the 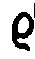 ⁰ cells where the values initially decline from 7 x 10⁻⁸ to 6 x 10⁻⁸ mol/l and then increase to 1.0 - 1.5 x 10⁻⁷ mol/l.

### Example 5

### Comparative morphological studies with confocal microscopy

The morphology of the mitochondria in the denoted cell lines was studied with confocal microscopy using MitoTracker Red CMXRos to stain the organelles (Fig. 6). The mitochondria of 143B.TK-wild type cells (Fig. 6A1) exist as reticular network evenly distributed within the cell. The prevalent mitochondrial Structure was elongated and rod shaped. In the 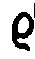 ⁰ form of these cells (Fig. 6A2, A3), the network structure appears disrupted, yielding a distribution of small individual mitochondrial units. This fragmentation is highlighted in the higher magnification images. Furthermore, some of these single organelles seemed to be swollen, this result could not be observed in the wild type.

These observations of an interconnected mitochondria network in wild type cells in contrast to punctated mitochondria in 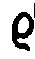 ⁰ cells constitute typical changes of mitochondrial morphology due to a loss of mitochondrial DNA (Gilkerson et al. (2000) FEBS Lett., 474, 1-4; Rizzuto et al. (1998) Science., 280, 1763-1766).

### Example 6

### Comparative morphological studies with transmission electron microscopy (TEM)

Even more morphological changes of the 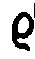 ⁰ mitochondria could be detected by ultrastructural studies (Fig. 7). Fig- 7A1 details a highly interconnected mitochondrial network structure in the wild type. The cross-section through the mitochondrial reticulum in normal 143B.TK⁻ cells showed a distinct outer and inner membrane with electron dense matrix full of regular arranged cistae sttuctures. In contrast the TEM images of the 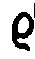 ⁰ cells (Fig. 7A2, A3) show an apparent altered morphology of the mitochondria. The network is degraded in single mitochondrial units, often with swollen appearance. The matrix seems to be electron empty probably due to a dilution of tube matrix by increasing influx of water that also explains the swollen appearance. The mitochondria in 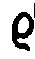 ⁰ cells still exhibit the distinct outer and inner membranes seen in normal cells; but the cristae displayed gross changes of structure. Most cristae appear curved or as concentric rings consisting of two membranes in close contact, an appearance denoted as "fuzzy onions" ( Hales and Fuller (1997) Cell., 90, 121-129; Halestrap (1989) Biochim. Biophys. Acta, 973, 355-382). Additionally, these membranous rings showed only few contact sites with the mitochondrial border membrane. It could not be distinguished whether these double membranes consist of two inner membranes or one outer and one inner membrane.

## Claims

1. An in-vitro method for the generation of a 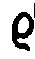 ⁰ cell comprising the following steps:
(a) transfecting mtDNA containing cells with an expression vector containing a gene encoding a fusion protein comprising a mitochondrial targeting sequence (MTS) and a restriction endonuclease operatively linked to a suitable promoter, wherein said restriction endonuclease cleaves the mtDNA 3 to 10 times;
(b) culturing the transfected cells over a sufficient period of time; and
(c) selecting 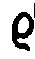 ⁰ cells.

2. The method of claim 1, wherein said restriction endonuclease is AflII, BamHI, BelI, EcoRI, HaeIII, HindII, HindIII, NdeI, PvuII or SpeI.

3. The method of claim 1 or 2, wherein said cells are animal cells.

4. The method of claim 3, wherein said animal cells are mammalian cells.

5. The method of any one of claims 1 to 4, wherein said promoter is the CMV, immediate early (IE) promoter, the Rous sarcoma virus (RSV) LTR or the SV40 virus early promoter.

6. The method of any one of claims 1 to 5 wherein said expression vector is a vector for animal cells.

7. The method of claim 6, wherein said vector is a human papova viral based vector (BKV), an SV40 derived vector, a vaccinia derived vector, an adeno viral derived vector, a baculo viral vector, or a retroviral derived vector.

8. The method of anyone of claims 1 to 7, wherein said MTS targets to the mitochondrial matrix.

9. The method of claim 8, wherein said MTS is a targeting peptide derived from the cytochrome c oxidase subunit 8 (OOX VIII).

10. The method of any one of claims 1 to 9, wherein said fusion protein furthermore comprises a detectable polypeptide.

11. The method of claim 10, wherein said detectable polypeptide is a fluorescence protein.

12. The method of claim 11, wherein said fluorescence protein is GFP or EGFP.

13. The method of any one of claims 1 to 12, wherein the 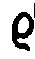 ⁰ cells are selected by FACS analysis, metabolic testing and/or genetic testing.

14. The method of claim 13, wherein said genetic testing is PCR or Southern Blot analysis.

## Patentansprüche

1. In-vitro-Verfahren zur Erzeugung einer 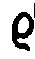 ⁰-Zelle, die folgenden Schritte umfassend:
(a) Transfizieren von mtDNA enthaltenden Zellen mit einem Expressionsvektor, der ein Gen enthält, das ein Fusionsprotein codiert, welches eine mitochondriale Targeting-Sequenz (MTS) und eine Restriktions-Endonuklease enthält, die operativ an einen geeigneten Promoter gebunden ist, wobei die Restriktions-Endonuklease die mtDNA 3 bis 10 Mal aufspaltet;
(b) Heranzüchten der transfizierten Zellen über einen ausreichenden Zeitraum; und
(c) Auswählen von 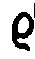 ⁰-Zellen.

2. Verfahren nach Anspruch 1, wobei die Restriktions-Endonuklease AflII, BamHI, BclI, EcoRI, HaeIII, HindII, HindIII, NdeI, PvuII oder SpeI ist.

3. Verfahren nach Anspruch 1 oder 2, wobei es sich bei den Zellen um tierische Zellen handelt.

4. Verfahren nach Anspruch 3, wobei es sich bei den tierischen Zellen um Säugetierzellen handelt.

5. Verfahren nach einem der Ansprüche 1 bis 4, wobei der Promoter der unmittelbare frühe (IE) Promoter von CMV, der Rous Sarkoma-Virus (RSV) LTR oder der frühe Promoter des SV 40-Virus ist.

6. Verfahren nach einem der Ansprüche 1 bis 5, wobei der Expressionsvektor ein Vektor für tierische Zellen ist.

7. Verfahren nach Anspruch 6, wobei der Vektor ein menschlicher Papova-Virus basierter Vektor (BKV), ein von SV40 abgeleiteter Vektor, ein von Kuhpocken abgeleiteter Vektor, ein vom Adenovirus abgeleiteter Vektor, ein vom Baculo-Virus abgeleiteter Vektor, oder ein retroviraler abgeleiteter Vektor ist.

8. Verfahren nach einem der Ansprüche 1 bis 7, wobei die MTS auf die mitochondriale Matrix abzielt.

9. Verfahren nach Anspruch 8, wobei die MTS ein zielgerichtet fungierendes Peptid ist, das aus der Cytochrom-C-Oxidase Untereinheit 8 (COX VIII) abgeleitet ist.

10. Verfahren nach einem der Ansprüche 1 bis 9, wobei das Fusionsprotein darüber hinaus ein detektierbares Polypeptid umfasst.

11. Verfahren nach Anspruch 10, wobei das detektierbare Polypeptid ein Fluoreszenzprotein ist.

12. Verfahren nach Anspruch 11, wobei das Fluoreszenzprotein GFP oder EGFP ist.

13. Verfahren nach einem der Ansprüche 1 bis 12, wobei die 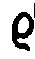 ⁰-Zellen durch eine FACS-Analyse, metabolische Untersuchung und/oder genetische Untersuchung ausgewählt werden.

14. Verfahren nach Anspruch 13, wobei die genetische Untersuchung eine PCR- oder Southern-Blot-Analyse ist.

## Revendications

1. Procédé in vitro pour la génération d'une cellule ρ°, comprenant les étapes suivantes :
a) transfecter des cellules contenant de l'ADN mitochondrial (mtDNA) avec un vecteur d'expression contenant un gène codant une protéine de fusion comprenant une séquence de ciblage mitochondrial (MTS) et une endonucléase de restriction liée de façon effective à un promoteur adéquat, pour lequel ladite endonucléase de restriction divise le mtDNA de 3 à 10 fois ;
b) effectuer une culture des cellules transfectées pendant une période de temps suffisante ; et
c) sélectionner des cellules ρ°.

2. Procédé selon la revendication 1, pour lequel ladite endonucléase de restriction est une AflII, une BamHI, une EcoRI, une HaeIII, une HindII, une HindIII, une Ndel, une PvuII ou une Spel.

3. Procédé selon la revendication 1 ou 2, pour lequel lesdites cellules sont des cellules animales.

4. Procédé selon la revendication 3, pour lequel lesdites cellules animales sont des cellules de mammifères.

5. Procédé selon l'une quelconque des revendications 1 à 4, pour lequel ledit promoteur est le promoteur précoce immédiat (IE) du CMV, le LTR du virus du sarcome de Rous (RSV) ou le promoteur précoce du virus SV40.

6. Procédé selon l'une quelconque des revendications 1 à 5, pour lequel ledit vecteur d'expression est un vecteur pour des cellules animales.

7. Procédé selon la revendication 6, pour lequel ledit vecteur un vecteur à base de papovavirus humain (BKV), un vecteur dérivé du SV40, un vecteur dérivé d'une vaccine, un vecteur dérivé d'adénovirus, un vecteur baculoviral ou un vecteur dérivé d'un rétrovirus.

8. Procédé selon l'une quelconque des revendications 1 à 7, pour lequel ledit MTS cible la matrice mitochondriale.

9. Procédé selon la revendication 8, pour lequel ledit MTS est un peptide cibleur dérivé de la sous-unité 8 du cytochrome c oxydase (OOX VIII).

10. Procédé selon l'une quelconque des revendications 1 à 9, pour lequel ladite protéine de fusion comprend en outre un polypeptide détectable.

11. Procédé selon la revendication 10, pour lequel ledit polypeptide détectable est une protéine de fluorescence.

12. Procédé selon la revendication 11, pour lequel ladite protéine de fluorescence est une GFP ou une EGFP.

13. Procédé selon l'une quelconque des revendications 1 à 12, pour lequel les cellules ρ° sont sélectionnées par une analyse FACS, un essai métabolique et/ou un essai génétique.

14. Procédé selon la revendication 13, pour lequel ledit essai génétique est une analyse PCR ou d'immunotransfert.
